# EUROPEAN PATENT SPECIFICATION

(11) **EP 4 203 875 B1**
(45) Date of publication and mention of the grant of the patent: **10.09.2025**
(21) Application number: 21759401.9
(22) Date of filing: 03.08.2021
(51) Int. Cl.: A61F 9/008, A61B 90/00

(54) **LASER SURGICAL SYSTEM FOR CREATING A MARKER IN AN EYE**
CHIRURGISCHES LASERSYSTEM ZUR ERZEUGUNG EINES MARKERS IN EINEM AUGE
SYSTÈME CHIRURGICAL LASER DE CRÉATION D'UN MARQUEUR DANS UN OEIL

(30) Priority: 26.08.2020 US 202063070775 P
(43) Date of publication of application: 05.07.2023
(73) Proprietor: Alcon Inc., 1701 Fribourg (CH)
(72) Inventor: ABRAHAM, Mario, 91058 Erlangen (DE)
(74) Representative: Mooser, Sebastian Thomas
(86) International application number: PCT/IB2021/057118
(87) International publication number: WO 2022/043799

(56) References cited:
- EP-A1- 1 252 872
- WO-A1-2013/096348
- WO-A2-2009/059251
- US-A- 4 848 340
- US-A1- 2012 078 240

## Description

### TECHNICAL FIELD

The present disclosure relates generally to laser surgical systems and methods, and more particularly to laser surgical systems and methods for creating a marker in an eye.

### BACKGROUND

Certain ophthalmic laser surgical systems generate a pulsed laser beam to perform a surgical procedure on an eye. In some procedures, the laser beam creates photodisruptions at specific points in the eye according to a treatment pattern. The laser beam should be properly aligned with the eye throughout the procedure to create photodisruptions that precisely match the pattern.

A patient interface (PI) is usually used to hold the eye in position during the procedure. The patient interface is typically affixed to the eye by a vacuum to secure the eye in place to facilitate proper alignment of the eye with the treatment pattern during the procedure.
US 2012/078240 A1 discloses an ultrashort pulsed laser instrument used to perform refractive surgery. The disclosure operates in ablative and incisional modalities. In the ablative mode, spiral ablation disks consisting of individual laser pulses are produced at high scanning speeds. Ablation profile may be produced in cornea by stacking and arranging multiple ablation disks to produce a specified shape change. Placement of ablation disks is assisted by an optical tracking and control system that compensates for eye motion. A preferred embodiment allows for ablative corrections to be performed on non-planar posterior surface of a laser cut flap affixed to registration platen, thereby avoiding exposing the eye interior to high radiant power. Laser cut and contrast agent dyed fiduciary marks may serve as reference features for the optical tracking system. Incisional procedures, such as corneal flaps for LASIK, may also be performed.

### BRIEF SUMMARY

The invention is defined in the appended claims.

In certain embodiments, an ophthalmic surgical system for creating a marker in a cornea for a surgical procedure includes controllable components, a camera, and a computer. The controllable components include a laser source, a scanner, and an objective. The laser source generates a laser beam having ultrashort pulses. The scanner transversely and longitudinally directs a focal point of the laser beam. The objective focuses the focal point through a patient interface towards the eye. The camera images movement of the eye. The marker has a shape indicating rotational movement of the eye. The computer creates the marker by: instructing the scanner to transversely and longitudinally direct the focal point towards a peripheral region of the cornea; and instructing one or more of the controllable components to create the marker in the peripheral region of the cornea. The computer also determines that movement of the marker is in an alert range indicating an unacceptable amount of movement, and provides one or more notifications in response to determining that the movement of the marker is in the alert range.

Embodiments may include none, one, some, or all of the following features:
* The shape of the marker is selected from one or more of the following: a polygon, a line, a plurality of lines, a plurality of lines intersecting at a point, a plurality of lines intersecting at a plurality of points, a circle with a line, an oval, and one or more alphanumeric characters.
* The computer identifies a marker corresponding to the surgical procedure and creates the identified marker in the cornea. In certain embodiments, if the surgical procedure is a lenticule extraction procedure, the computer identifies that the marker is external to and proximate to an outer boundary of a lenticule to be extracted, and creates the marker external to and proximate to the outer boundary. In certain embodiments, if the surgical procedure is a flap creation procedure, the computer identifies that the marker is external to and proximate to an outer boundary of a flap to be created, and creates the marker external to and proximate to the outer boundary. In certain embodiments, if the surgical procedure is a cataract removal procedure, the computer identifies that the marker outlines a lens capsule of the eye, and creates the marker that outlines the lens capsule of the eye.
* A notification may be an audio notification.
* A notification may be a visual notification.
* The alert range comprises a plurality of non-overlapping subset alert ranges that form a partition of the alert range. The computer provides a plurality of notifications. Each notification corresponds to a subset alert range, where a first notification for a first subset alert range is distinct from a second notification for a second subset alert range. In certain embodiments, the first notification has a first visual characteristic that is distinct from a second visual characteristic of the second notification. In certain embodiments, the first notification has a first audio characteristic that is distinct from a second audio characteristic of the second notification. In certain embodiments, a subset alert range is a terminating alert range, and the computer terminates the surgical procedure in response to determining that the movement of the marker is in the terminating alert range. In certain embodiments, the ophthalmic surgical system includes a display screen. The computer displays a notification icon comprising alert levels on the display screen, where an alert level corresponds to a subset alert range, and visually emphasizes an alert level in response to determining that the movement of the marker is in the corresponding subset alert range.
* The computer determines the alert range corresponding to the surgical procedure. In certain embodiments, if the surgical procedure is a lenticule extraction procedure, the alert range has a maximum acceptable distance of 50 to 150 microns. In certain embodiments, if the surgical procedure is a flap creation procedure, the alert range has a maximum acceptable distance of 100 to 500 microns. In certain embodiments, if the surgical procedure is a cataract removal procedure, the alert range has a maximum acceptable distance of 100 to 500 microns.

A method for creating a marker in a cornea of an eye for a surgical procedure is described for illustrative purposes only and does not form a part of the claimed subject-matter. The method includes creating, by a computer, the marker in the cornea. The marker has a shape that can indicate rotational movement of the eye. The computer instructs a scanner to transversely and longitudinally direct a focal point of a laser beam towards a peripheral region of the cornea, and instructs one or more of a set of controllable components to create the marker in the peripheral region of the cornea. The set of controllable components comprises a laser source configured to generate the laser beam, the scanner configured to transversely and longitudinally direct the focal point of the laser beam, and an objective configured to focus the focal point through a patient interface towards the eye. The computer also determines that movement of the marker is in an alert range, the alert range indicating an unacceptable amount of movement of the marker, and provides one or more notifications in response to determining that the movement of the marker is in the alert range.

Embodiments may include none, one, some, or all of the features described above with respect to the system and/or none, one, some, or all of the following features:
* The method further includes identifying the marker corresponding to the surgical procedure, and creating the identified marker in the cornea.
* The alert range comprises a plurality of non-overlapping subset alert ranges that form a partition of the alert range. The method further includes: providing a plurality of notifications, each notification corresponding to a subset alert range, a first notification for a first subset alert range distinct from a second notification for a second subset alert range; displaying a notification icon comprising a plurality of alert levels on a display screen, at least one alert level corresponding to a subset alert range; and visually emphasizing an alert level in response to determining that the movement of the marker is in the corresponding subset alert range.

### BRIEF DESCRIPTION OF THE DRAWINGS

FIGURE 1 illustrates an example of an ophthalmic surgical system configured to create a marker in the cornea of an eye according to certain embodiments;
FIGURES 2A and 2B illustrate an example of a marker created in an eye;
FIGURE 3 illustrates a marker that includes circles with a line;
FIGURE 4 illustrates a marker that includes alphanumeric characters;
FIGURE 5 illustrates a marker that includes lines;
FIGURE 6 illustrates a marker that includes lines and a circle;
FIGURE 7 illustrates an example of a traffic light notification icon that may be used by the system of FIGURE 1;
FIGURE 8 illustrates an example of a meter notification icon that may be used by the system of FIGURE 1; and
FIGURE 9 illustrates an example of a method for creating a marker in an eye that may be performed by the system of FIGURE 1.

### DESCRIPTION OF EXAMPLE EMBODIMENTS

Referring now to the description and drawings, example embodiments of the disclosed apparatuses, systems, and methods are shown in detail. The description and drawings are not intended to be exhaustive or otherwise limit the claims to the specific embodiments shown in the drawings and disclosed in the description. Although the drawings represent possible embodiments, the drawings are not necessarily to scale and certain features may be simplified, exaggerated, removed, or partially sectioned to better illustrate the embodiments.

In certain ophthalmic surgical procedures, the eye is coupled to a surgical system with a patient interface (PI) in order to properly align the eye with a treatment pattern. An issue may arise in that the eye might move relative to the patient interface, and thus relative to the treatment pattern. For example, a nervous patient may vigorously move their eyes, creating a shear force strong enough for the eye to move relative to the patient interface or even become disconnected from the interface. As another example, an inexperienced surgeon may inadvertently move the eye relative to the patient interface or may fail to properly connect the eye to the patient interface such that the eye moves relative to the interface. Movement of the eye relative to the patient interface may cause the eye to be misaligned with the treatment pattern. Embodiments described herein may address this issue.

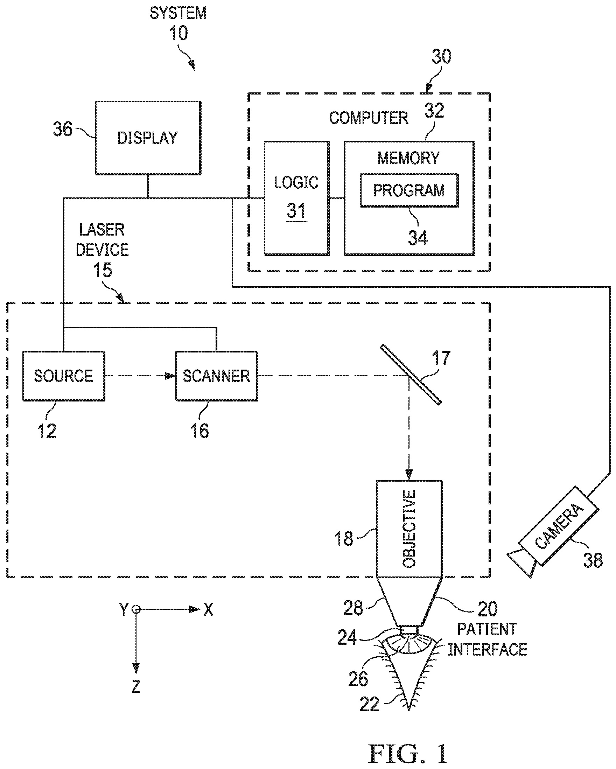

FIGURE 1 illustrates an example of an ophthalmic surgical system 10 configured to create a marker in the cornea of an eye 22 according to certain embodiments. In the embodiments, system 10 creates the marker in the cornea, tracks the movement of the marker, and provides a notification if the movement is in an alert range indicating that the eye has moved relative to the patient interface. Accordingly, system 10 may be used to address the issue of eye 22 moving relative to the patient interface and becoming misaligned with the treatment pattern.

In the illustrated example, system 10 includes a laser device 15, a patient interface 20, a camera 38, and a control computer 30, coupled as shown. Laser device 15 includes controllable components, such as a laser source 12, a scanner 16, one or more optical elements 17, and/or a focusing objective 18, coupled as shown. Patient interface 20 includes a contact portion 24 (with an abutment face 26) and a sleeve 28 coupled as shown. Computer 30 includes logic 31, a memory 32 (which stores a computer program 34), and a display 36, coupled as shown.

As an overview, system 10 can create and track the marker according to the following example of operation. Laser source 12 generates a laser beam with ultrashort pulses. Scanner 16 transversely and longitudinally controls a focal point of the laser beam. Objective 18 focuses the focal point through patient interface 20 towards eye 22. Camera 38 images movement of eye 22. Computer 30 creates the marker in the cornea by instructing scanner 12 to direct the focal point towards a peripheral region of the cornea and by instructing the controllable components to create the marker in the peripheral region. Computer 30 then determines whether the movement of the marker is in an alert range describing unacceptable movement of the marker. If so, computer 30 provides one or more notifications that the movement is in the alert range.

Turning to the parts of system 10, laser source 12 generates a laser beam with ultrashort pulses. An ultrashort pulse refers to a light pulse that has a duration that is less than a nanosecond, such as on the order of picoseconds, femtoseconds, or attoseconds. The laser beam may have any suitable wavelength, such as a wavelength in the range of 300 to 1500 nanometers (nm), e.g., a wavelength in the range of 300 to 650, 650 to 1050, 1050 to 1250, and/or 1250 to 1500 nm, such as 340 to 350 nm, e.g., 347 nm ± 1 nm. The focal point of the laser beam may create a laser-induced optical breakdown (LIOB) in tissue (e.g., the cornea) to yield a photodisruption in the tissue. The laser beam may be precisely focused to yield precise photodisruptions, which may reduce or avoid unnecessary destruction of other tissue.

Scanner 16 transversely and longitudinally directs the focal point of the laser beam. The longitudinal direction refers to the direction of the laser beam propagation, also known as the z-direction. The transverse direction refers to directions orthogonal to the direction of beam propagation, also known as the xy-plane. In certain embodiments, abutment face 26 of patient interface 20 is selected as the xy-plane at z = 0.

Scanner 16 may transversely direct the laser beam in any suitable manner. For example, scanner 16 may include a pair of galvanometrically-actuated scanner mirrors that can be tilted about mutually perpendicular axes. As another example, scanner 16 may include an electro-optical crystal that can electro-optically steer the laser beam. Scanner 16 may longitudinally direct the laser beam in any suitable manner. For example, scanner 16 may include a longitudinally adjustable lens, a lens of variable refractive power, or a deformable mirror that can control the z-position of the beam focus. The components of scanner 16 may be arranged in any suitable manner along the beam path, e.g., in the same or different modular units.

One (or more) optical elements 17 direct the laser beam towards focusing objective 18. An optical element 17 can act on (e.g., transmit, reflect, refract, diffract, collimate, condition, shape, focus, modulate, and/or otherwise act on) a laser beam. Examples of optical elements include a lens, prism, mirror, diffractive optical element (DOE), holographic optical element (HOE), and spatial light modulator (SLM). In the example, optical element 17 is a mirror. Focusing objective 18 focuses the focal point of laser beam through the patient interface 20 towards a point of eye 22. In the example, focusing objective 18 is an objective lens, e.g., an f-theta objective.

Patient interface 20 interfaces with the cornea of eye 22 to couple eye 22 to laser device 15. In the example, patient interface 20 has sleeve 28 coupled to contact portion 24. Sleeve 28 detachably couples to focusing objective 18. Contact portion 24 may be translucent or transparent to the laser beam and has an abutment face 26 that interfaces with the cornea. In certain embodiments, abutment face 26 is planar and forms a planar area on the cornea, which may define the xy-plane. In other embodiments, abutment face 26 need not be planar, e.g., may be convex or concave.

Camera 38 records images of the movement of eye 22, which includes movement of the marker created in eye 22. Examples of camera 38 include a video, optical coherence tomography (OCT), or eye-tracking camera. Camera 38 delivers image data, which represent recorded images of the eye 22, to computer 30. Computer 30 carries out image processing on the image data to determine movement of the marker. The image processing includes recognizing the marker in the recorded images, determining the position of the marker, and comparing the positions of the marker imaged at different times to determine the movement of the marker.

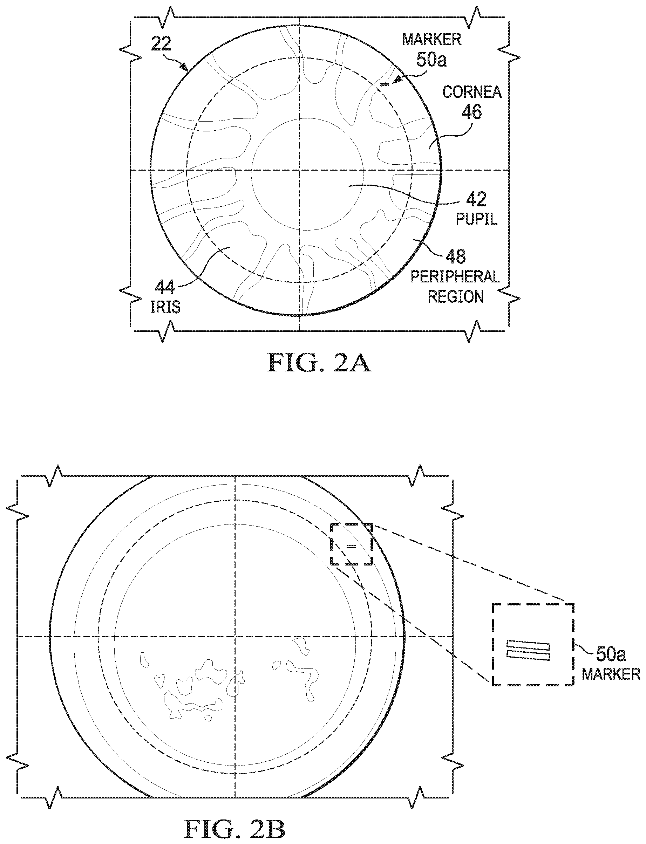

Computer 30 controls controllable components (e.g., laser source 12, scanner 16, optical elements 17, and/or focusing objective 18) in accordance with computer program 34. Computer program 34 includes computer code that instructs the controllable components to focus the laser beam at a region of the cornea and to photodisrupt at least a portion of the region to create a marker in the cornea. The marker is temporary and disappears on its own. Markers are described in more detail with reference to FIGURES 2A to 6.

FIGURES 2A and 2B illustrate an example of a marker 50 (50a) created in eye 22. FIGURE 2A shows eye 22 with a pupil 42, an iris 44, a lens (not shown), and a cornea 46 with a peripheral region 48. In general, iris 44 and cornea 46 each have an outer diameter in the range of 10 to 13 millimeters (mm). Cornea 46 has a peripheral region 48, which is an annular region of cornea 46 closest to the outer perimeter of cornea 46. Peripheral region 48 may have an outer radius of Rc and an inner radius of Rc x q, where Rc represents the outer radius of cornea 46, and q is any suitable percentage, e.g., 50 to 60, 60 to 70, 70 to 80, 80 to 90, and/or 90 to 95 percent.

The inner radius may be determined in accordance with the surgical procedure. In some cases, the inner radius may be such that peripheral region 48 does not interfere with (e.g., is outside of) the treatment area of the procedure. For example, a typical lenticule diameter is approximately 5 to 7 mm, and a typical flap diameter is approximately 7 to 10 mm. The inner radius may be selected such that peripheral region 48 is outside of the lenticule or flap region. In other cases, the inner radius may be such that peripheral region 48 includes or forms a border for a surgical procedure. For example, a marker 50 may be used to outline a lens capsule, which has a diameter of approximately 9 to 10 mm. The inner radius may be selected to be less than or to match the radius of the lens capsule.

Marker 50 may have any suitable size and shape. In certain embodiments, marker 50 has a size and shape that can readily indicate translation and/or rotation of eye 22. For example, marker 50 has size sufficiently large such that camera 38 can readily detect the movement of marker 50, but sufficiently small to minimize photodisruptions of eye 22. As another example, marker 50 has a shape such that camera 38 can detect translation and/or rotation of marker 50.

Marker 50 may have any suitable location in peripheral region 48. In certain embodiments, the location of marker 50 may be determined according to the surgical procedure, such as relative to the treatment zone and/or field of view of the procedure. For example, in a lenticule extraction procedure, the treatment zone, i.e., the lenticule, may be up to approximately 8 millimeters (mm) in diameter, and the field of view may be approximately 12 mm in diameter. Marker 50 may be outside of and proximate to (e.g., within 0.010 to 2 mm, such as 0.015 mm) the outer boundary of the lenticule, but within the field of view. As another example, for a flap creation procedure, marker 50 may be outside of and proximate to the outer boundary of the flap. As yet another example, for a cataract removal procedure, marker 50 may outline the lens capsule of the eye.

Examples of shapes include: a polygon (e.g., a triangle or square), a line, a plurality of lines (e.g., an equal sign), a plurality of lines intersecting at a point (e.g., a plus sign, a cross, or an asterisk), a plurality of lines intersecting at a plurality of points (e.g., a star), a circle with a line, an oval, and one or more alphanumeric characters (e.g., text with letters of any language, numbers, and/or symbols). In the illustrated example, marker 50a is shaped like an equal sign. FIGURE 2A shows marker 50a with zero rotation, such that the equal sign is horizontal. FIGURE 2B shows marker 50a that has been rotated 5 degrees, such that the equal sign is tilted at an angle.

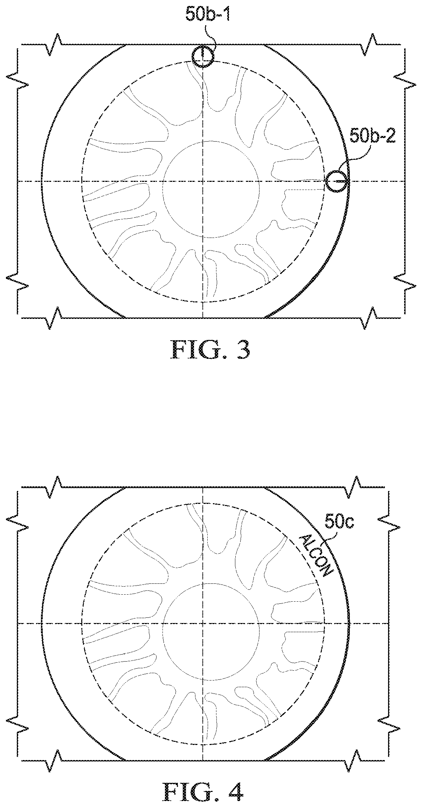

FIGURES 3 to 6 illustrate different types of markers 50. FIGURE 3 illustrates a marker 50 that includes markers 50b (50b-1, 50b-2), where each marker 50b is a circle with a line. The line of marker 50b-1 is aligned with a vertical line through a center of eye 22 (e.g., pupil center, eye apex, or eye vertex), and the line of marker 50b-2 is aligned with a horizontal line through the center.

FIGURE 4 illustrates a marker 50c that includes alphanumeric characters. In certain embodiments, the characters may provide information, e.g., a time stamp, a patient identifier, or a company name.

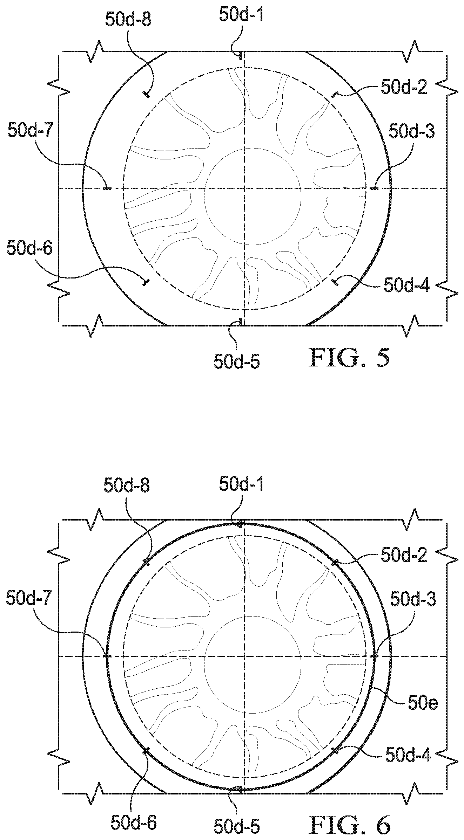

FIGURE 5 illustrates a marker 50 that includes markers 50d (50d-1 to 50d-8). Each marker 50d is a line that is aligned with a line that passes through the center of eye 22. For example, markers 50d-1 and 50d-5 are aligned with a vertical line through the center, and markers 50d-3 and 50d-7 are aligned with a horizontal line through the center.

FIGURE 6 illustrates a marker 50 that includes markers 50d and a marker 50e. Marker 50e is a circle that may provide a border for a treatment area, e.g., provide an outline for a lenticule, flap, or lens capsule.

Returning to FIGURE 1, recall that computer 30 instructs the controllable components of laser device 15 to create marker 50. In certain embodiments, computer 30 determines the type of marker 50 to create by identifying the marker 50 corresponding to the surgical procedure. In the embodiments, particular features of a marker 50 (e.g., size, shape, location, and/or position) may be more appropriate for a specific surgical procedure. For example, computer 30 may determine that: (1) for a lenticule extraction procedure, marker 50 may be outside of and proximate to the outer boundary of the lenticule; (2) for a flap creation procedure, marker 50 may be outside of and proximate to the outer boundary of the flap; and/ or (3) for a cataract removal procedure, marker 50 may outline the lens capsule of the eye.

Also recall that computer 30 determines when the movement of marker 50 is in an alert range and provides a notification in response to the determination. An alert range describes an unacceptable amount of movement of marker 50, which represents an unacceptable amount of movement of eye 22. An unacceptable movement may be movement that misaligns eye 22 with the treatment pattern such that a notification should be provided and/or the procedure should be terminated. An alert range may be expressed as movement of a distance d greater than a maximum acceptable distance Q, expressed in set notation as {d | d > Q}, where Q is any suitable number, e.g.., 50 to 500 microns.

In certain embodiments, the alert range comprises a plurality of non-overlapping subset alert ranges that form a partition of the alert range. A partition of a set is collection of disjoint subsets of the set where the union of the subsets equals the set. For example, the alert range {d | d > Q} may be partitioned into {*d* | *Q*1 *< d* ≤ *Q*2, *Q*2 *< d* ≤ *Q*3, *..., Qn < d*}, where Q = Q1. A subset alert range closer to the maximum acceptable distance Q indicates less misalignment than a subset alert range farther away from the maximum acceptable distance Q. Accordingly, a movement in a subset alert range closer to the maximum acceptable distance Q may be less urgent than a movement in one farther away. In certain embodiments, a subset alert range may be a terminating alert range, which represents unacceptable movement such that the procedure should be terminated.

In certain embodiments, computer 30 determines the alert range corresponding to the surgical procedure. Certain procedures (e.g., creating a lenticule) may require more precision than other procedures (e.g., creating a flap). For example, a lenticule has a shape with refractive properties, so the lenticular incision should be precisely aligned with the treatment pattern. A flap typically has a flat bed incision with no refractive properties, so the alignment need not be as precise. Accordingly, the maximum acceptable distance Q for the alert range may be stricter for a procedure that require more precision. For example, for a lenticule extraction procedure, the maximum acceptable distance Q may be in the range of 50 to 150 microns. As another example, for a flap creation procedure, the maximum acceptable distance Q may be in the range of 100 to 500 microns. As yet another example, for a cataract removal procedure, the maximum acceptable distance Q may be in the range of 50 to 500.

Computer 30 may provide any suitable notification, e.g., audio and/or visual notification. Examples of audio notifications include beeps and spoken words that may be provided via an interface such as an electronic speaker. Examples of visual notifications include lights and graphical icons that may be displayed on an interface such as a display 36 (e.g., a display screen).

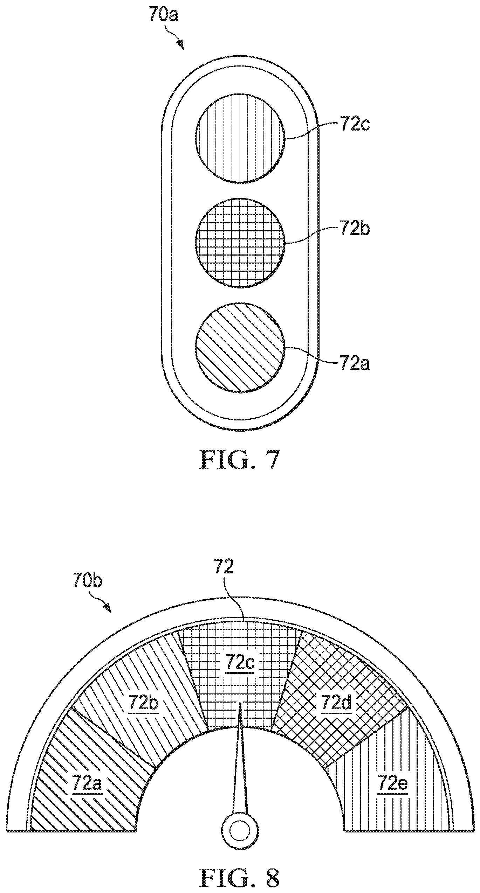

In certain embodiments, computer 30 provides a plurality of notifications for a plurality of subset alert ranges. In some instances, each notification corresponds to a particular subset alert range, and computer 30 provides different notifications for different subset alert ranges, e.g., a first notification for a first subset alert range is distinct from a second notification for a second subset alert range. For example, the first notification may have a first visual characteristic that is distinct from a second visual characteristic of the second notification. As another example, the first notification may have a first audio characteristic that is distinct from a second audio characteristic of the second notification. In certain embodiments, computer 30 terminates the surgical procedure in response to determining that the movement of the marker is in a terminating alert range. Examples of visual notification icons for multiple subset alert ranges are described with reference to FIGURES 7 and 8.

FIGURE 7 illustrates an example of a traffic light notification icon 70 (70a) that may be used by system 10 of FIGURE 1. A notification icon 70 is a graphical element (which may be presented via display 36) that provides a notification when the movement of marker 50 reaches an alert range. In certain embodiments, notification icon 70 includes alert levels 72 (72a, 72b, 72c). An alert level 72 may correspond to a subset alert range. If the movement of marker 50 falls into a subset alert range, the icon 70 may visually emphasize (e.g., light up, point to, blink, outline, or otherwise emphasize) the corresponding alert level 72.

In the illustrated example, alert level 72a is a green light, alert level 72b is a yellow light, and alert level 72c is a red light. When the movement falls into a subset alert range, icon 70 may emphasize (e.g., light up or make brighter) the corresponding light. Green alert level 72a may correspond to no movement of marker 50 or movement that has not reached an alert level, i.e., the movement is in an acceptable range. Yellow alert level 72b may correspond to the subset alert range closest to the minimum alert value, i.e., the movement is not acceptable, but not to the point that requires terminating the procedure. Red alert level 72c may correspond to a subset alert range that is a terminating alert range, i.e., the movement requires terminating the procedure.

FIGURE 8 illustrates an example of a meter notification icon 70 (70b) that may be used by system 10 of FIGURE 1. Notification icon 70b includes alert levels 72 (72a to 72e). In the illustrated example, alert level 72a is green; alert level 72b is yellowish green; alert level 72c is yellow; 72d is orange; and alert level 72e is red.

Certain alert levels 72 may correspond to a subset alert range. When the movement of marker 50 falls into a subset alert range, icon 70 may emphasize (e.g., point to) the corresponding level 72. Green alert level 72a may correspond to no movement of marker 50 or movement that has not reached an alert level, i.e., the movement is in the acceptable range. Yellowish green alert level 72b may correspond to the subset alert range closest to the minimum alert value, i.e., the movement is not acceptable, but close to acceptable. Yellow alert level 72c may correspond to the next closest subset alert range, i.e., the movement is not acceptable, but not to the point that requires terminating the procedure. Orange alert level 72d may correspond to the next closest subset alert range, i.e., the movement is not acceptable and close to the point that requires terminating the procedure. Red alert level 72e may correspond to a subset alert range that is a terminating alert range, i.e., the movement requires terminating the procedure.

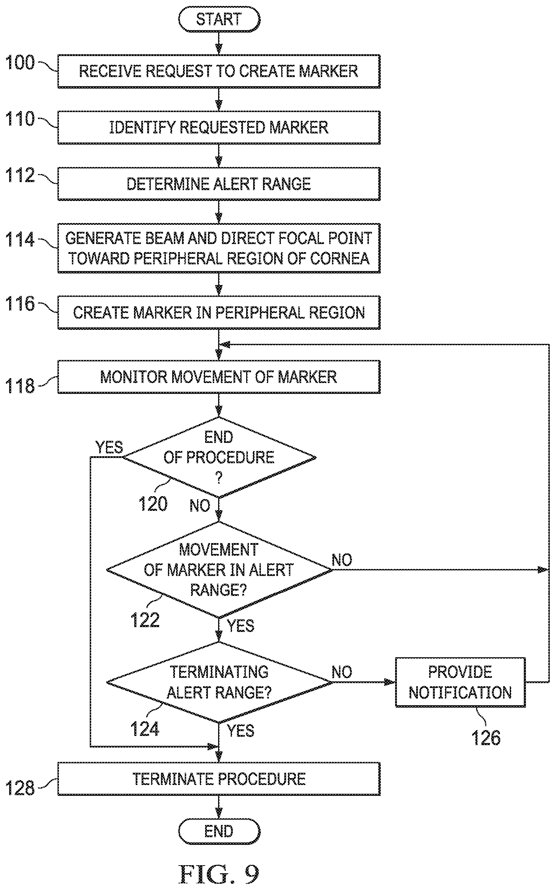

FIGURE 9 illustrates an example of a method for creating marker 50 in eye 22 that may be performed by system 10 of FIGURE 1. In certain embodiments, computer 30 performs the method by instructing parts of system 10 to perform the actions of the method. Marker 50 may be created during a surgical procedure, e.g., a lenticule extraction, flap creation, or a cataract removal procedure. The method starts at step 100, where computer 30 receives a request to create marker 50 in cornea 46 of eye 40. Marker 50 may have a shape that can indicate translational and/or rotational movement of eye 22. In certain embodiments, the request may be received from a user (e.g., surgeon) of system 10 or may be received from a computer program 34 for an automated surgical procedure.

Computer 30 identifies the requested marker 50 at step 110. In certain embodiments, computer 30 may identify a marker 50 that corresponds to the surgical procedure. For example, a lenticule extraction procedure may use a marker 50 that is external to and proximate to the outer boundary of the lenticule to be extracted. As another example, a flap creation procedure may use a marker 50 that is external to and proximate to the outer boundary of the flap to be created. As yet another example, a cataract procedure may use a marker 50 that outlines the lens capsule of the cataractous lens.

At step 112, computer 30 determines an alert range. In certain embodiments, computer 30 may determine an alert range that corresponds to the surgical procedure. For example, a lenticule extraction procedure may use an alert range with a maximum acceptable distance Q of 50 to 150 microns. As another example, a flap creation procedure may use an alert range with a maximum acceptable distance Q of 100 to 500 microns. As yet another example, a cataract procedure may use an alert range with a maximum acceptable distance Q of 50 to 500 microns.

Computer 30 instructs laser source 12 to generate a laser beam and instructs scanner 16 to direct the focal point of the beam towards peripheral region 48 of cornea 46 at step 114. The controllable components create marker 50 in peripheral region 48 at step 116. Computer 30 monitors the movement of marker 50 at step 118 using camera 38.

The surgical procedure may be completed at step 120. If the procedure has been completed, the method proceeds to step 128, where computer 30 terminates the procedure. If the procedure has not been completed, the method proceeds to step 122.

Computer 30 checks whether the movement of marker 50 is in the alert range at step 122. If the movement is not in the alert range, the method returns to step 118, where computer 30 continues to monitor the movement of marker 50. If the movement is in the alert range, the method proceeds to step 124.

At step 124, computer 30 checks whether the alert range is a terminating alert range. A terminating alert range indicates when the surgical procedure should be terminated. For example, the terminating range may indicate the eye is so misaligned that the treatment will not be effective. If the alert range is not a terminating alert range, the method proceeds to step 126.

At step 126, computer 30 provides a notification that the movement is in the alert range. In certain embodiments, the alert range has multiple non-overlapping subset alert ranges that form a partition of the alert range, and computer 30 provides a notification for each subset alert range. In certain cases, computer 30 provides distinct notifications for different subset ranges. For example, a first notification for a first subset alert range may be distinct from a second notification for a second subset alert range. The notifications may be visually distinct (e.g., the first notification has a first visual characteristic that is distinct from a second visual characteristic of the second notification) or aurally distinct (e.g., the first notification has a first audio characteristic that is distinct from a second audio characteristic of the second notification). The method then returns to step 118, where computer 30 continues to monitor the movement of marker 50.

If the alert range is a terminating alert range at step 124, the method proceeds to step 128, where computer 30 terminates the procedure. The method then ends.

A component (e.g., control computer 30) of the systems and apparatuses disclosed herein may include an interface, logic, and/or memory, any of which may include computer hardware and/or software. An interface (e.g., display 36) can receive input to the component and/or send output from the component, and is typically used to exchange information between, e.g., software, hardware, peripheral devices, users, and combinations of these. A user interface (e.g., a Graphical User Interface (GUI)) is a type of interface that a user can utilize to interact with a computer. Examples of interfaces include a display screen, touchscreen, keyboard, mouse, gesture sensor, microphone, and speakers.

Logic (e.g., logic 31) can perform operations of the component. Logic may include one or more electronic devices that process data, e.g., execute instructions to generate output from input. Examples of such an electronic device include a computer, processor, microprocessor (e.g., a Central Processing Unit (CPU)), and computer chip. Logic may include computer software that encodes instructions capable of being executed by the electronic device to perform operations. Examples of computer software include a computer program, application, and operating system.

A memory (e.g., memory 32) can store information and may comprise tangible, computer-readable, and/or computer-executable storage medium. Examples of memory include computer memory (e.g., Random Access Memory (RAM) or Read Only Memory (ROM)), mass storage media (e.g., a hard disk), removable storage media (e.g., a Compact Disk (CD) or Digital Video or Versatile Disk (DVD)), database, network storage (e.g., a server), and/or other computer-readable media. Particular embodiments may be directed to memory encoded with computer software.

Although this disclosure has been described in terms of certain embodiments, modifications (such as changes, substitutions, additions, omissions, and/or other modifications) of the embodiments will be apparent to those skilled in the art. Accordingly, modifications may be made to the embodiments without departing from the scope of the invention. For example, modifications may be made to the systems and apparatuses disclosed herein. The components of the systems and apparatuses may be integrated or separated, or the operations of the systems and apparatuses may be performed by more, fewer, or other components, as apparent to those skilled in the art.

## Claims

1. An ophthalmic surgical system (10) for creating a marker in a cornea of an eye (22) for a surgical procedure of a plurality of surgical procedures, comprising:
a plurality of controllable components comprising:
a laser source (12) configured to generate a laser beam having a plurality of ultrashort pulses;
a scanner (16) configured to transversely and longitudinally direct a focal point of the laser beam;
an objective (18) configured to focus the focal point through a patient interface (20) towards the eye (22); and
a camera (38) configured to image movement of the eye (22);
and a computer (30) configured to:
identify the marker corresponding to the surgical procedure, the surgical procedures comprising a cataract removal procedure, the marker comprising a marker that outlines a lens capsule of the eye (22);
create the marker in the cornea that outlines the lens capsule of the eye (22), the marker having a shape that can indicate rotational movement of the eye, by:
instructing the scanner (16) to transversely and longitudinally direct the focal point towards a peripheral region of the cornea;
instructing one or more of the controllable components to create the marker in the peripheral region of the cornea;
determine that movement of the marker is in an alert range, the alert range indicating an unacceptable amount of movement of the marker; and
provide one or more notifications in response to determining that the movement of the marker is in the alert range.

2. The ophthalmic surgical system (10) of Claim 1, wherein the shape of the marker is selected from one or more of the following: a polygon, a line, a plurality of lines, a plurality of lines intersecting at a point, a plurality of lines intersecting at a plurality of points, a circle with a line, an oval, and one or more alphanumeric characters.

3. The ophthalmic surgical system (10) of Claim 1, wherein:
another surgical procedure of the plurality of surgical procedures is a lenticule extraction procedure; and
the computer (30) is configured to:
identify a marker that is external to and proximate to an outer boundary of a lenticule to be extracted; and
create the marker external to and proximate to the outer boundary.

4. The ophthalmic surgical system (10) of Claim 1, wherein:
another surgical procedure of the plurality of surgical procedures is a flap creation procedure; and
the computer (30) is configured to:
identify a marker that is external to and proximate to an outer boundary of a flap to be created; and
create the marker external to and proximate to the outer boundary.

5. The ophthalmic surgical system (10) of Claim 1, wherein a notification of the one or more notifications is an audio notification.

6. The ophthalmic surgical system (10) of Claim 1, wherein a notification of the one or more notifications is a visual notification.

7. The ophthalmic surgical system (10) of Claim 1, wherein:
the alert range comprises a plurality of non-overlapping subset alert ranges that form a partition of the alert range; and
the computer (30) is configured to provide a plurality of notifications, each notification corresponding to a subset alert range, a first notification for a first subset alert range distinct from a second notification for a second subset alert range.

8. The ophthalmic surgical system (10) of Claim 7, wherein the first notification has a first visual characteristic that is distinct from a second visual characteristic of the second notification.

9. The ophthalmic surgical system (10) of Claim 7, wherein the first notification has a first audio characteristic that is distinct from a second audio characteristic of the second notification.

10. The ophthalmic surgical system (10) of Claim 7, wherein:
a subset alert range of the plurality of subset alert ranges is a terminating alert range; and
the computer (30) is configured to terminate the surgical procedure in response to determining that the movement of the marker is in the terminating alert range.

11. The ophthalmic surgical system (10) of Claim 7, wherein:
the ophthalmic surgical system (10) further comprises a display screen; and
the computer (30) is configured to:
display a notification icon comprising a plurality of alert levels on the display screen, at least one alert level corresponding to a subset alert range; and
visually emphasize the at least one alert level in response to determining that the movement of the marker is in the corresponding subset alert range.

12. The ophthalmic surgical system (10) of Claim 1, the computer (30) further configured to determine the alert range corresponding to a selected surgical procedure of the plurality of surgical procedures.

13. The ophthalmic surgical system (10) of Claim 12, wherein:
the selected surgical procedure is a lenticule extraction procedure; and
the alert range has a maximum acceptable distance of 50 to 150 microns.

14. The ophthalmic surgical system (10) of Claim 12, wherein:
the selected surgical procedure is a flap creation procedure; and
the alert range has a maximum acceptable distance of 100 to 500 microns.

15. The ophthalmic surgical system (10) of Claim 12, wherein:
the selected surgical procedure is a cataract removal procedure; and
the alert range has a maximum acceptable distance of 100 to 500 microns.

## Patentansprüche

1. Ophthalmisches chirurgisches System (10) zum Erzeugen eines Markers in einer Hornhaut eines Auges (22) für einen chirurgischen Eingriff von einer Vielzahl chirurgischer Eingriffe, umfassend:
eine Vielzahl von steuerbaren Komponenten, umfassend:
eine Laserquelle (12), die dazu ausgelegt ist, einen Laserstrahl mit einer Vielzahl von ultrakurzen Impulsen zu generieren;
einen Scanner (16), der dazu ausgelegt ist, einen Fokuspunkt des Laserstrahls in Querrichtung und in Längsrichtung zu richten;
ein Objektiv (18), das dazu ausgelegt ist, den Fokuspunkt durch eine Patientenschnittstelle (20) in Richtung des Auges (22) zu fokussieren; und
eine Kamera (38), die dazu ausgelegt ist, um Bewegung des Auges (22) abzubilden; und
einen Computer (30), der ausgelegt ist zum:
Identifizieren des Markers, der dem chirurgischen Eingriff entspricht, wobei die chirurgischen Eingriffe einen Kataraktentfernungseingriff umfassen, wobei der Marker einen Marker umfasst, der eine Linsenkapsel des Auges umreißt (22);
Erzeugen des Markers in der Hornhaut, der die Linsenkapsel des Auges (22) umreißt, wobei der Marker eine Form aufweist, die Rotationsbewegung des Auges angeben kann, durch:
Anweisen des Scanners (16), den Fokuspunkt in Querrichtung und in Längsrichtung auf eine peripheren Region der Hornhaut zu richten;
Anweisen einer oder mehreren der steuerbaren Komponenten, den Marker in der peripheren Region der Hornhaut zu erzeugen;
Bestimmen, dass sich die Bewegung des Markers in einem Alarmbereich befindet, wobei der Alarmbereich einen inakzeptablen Bewegungsbetrag des Markers angibt; und
Bereitstellen einer oder mehrerer Benachrichtigungen in Reaktion auf Bestimmen, dass sich die Bewegung des Markers im Alarmbereich befindet.

2. Ophthalmisches chirurgisches System (10) nach Anspruch 1, wobei die Form des Markers ausgewählt ist aus einem oder mehreren von einem Polygon, einer Linie, einer Vielzahl von Linien, einer Vielzahl von Linien, die sich an einem Punkt schneidet, einer Vielzahl von Linien, die sich an einer Vielzahl von Punkten schneidet, einem Kreis mit einer Linie, einem Oval und einem oder mehreren alphanumerischen Zeichen.

3. Ophthalmisches chirurgisches System (10) nach Anspruch 1, wobei:
ein anderer chirurgischer Eingriff der Vielzahl von chirurgischen Eingriffen ein Lentikel-Extraktionseingriff ist; und
der Computer (30) ausgelegt ist zum:
Identifizieren eines Markers, der sich außerhalb einer Außengrenze eines zu extrahierenden Lentikels und in der Nähe zu dieser befindet; und
Erzeugen des Markers außerhalb der Außengrenze und in der Nähe zu dieser.

4. Ophthalmisches chirurgisches System (10) nach Anspruch 1, wobei:
ein anderer chirurgischer Eingriff der Vielzahl von chirurgischen Eingriffen ein Flap-Erzeugungseingriff ist; und
der Computer (30) ausgelegt ist zum:
Identifizieren eines Markers, der sich außerhalb der Außengrenze eines zu erzeugenden Flaps und in der Nähe zu dieser befindet; und
Erzeugen des Markers außerhalb der Außengrenze und in der Nähe zu dieser.

5. Ophthalmisches chirurgisches System (10) nach Anspruch 1, wobei eine Benachrichtigung der einen oder mehreren Benachrichtigungen eine Audiobenachrichtigung ist.

6. Ophthalmisches chirurgisches System (10) nach Anspruch 1, wobei eine Benachrichtigung der einen oder mehreren Benachrichtigungen eine visuelle Benachrichtigung ist.

7. Ophthalmisches chirurgisches System (10) nach Anspruch 1, wobei:
der Alarmbereich eine Vielzahl von nicht-überlappenden Teilsätzen von Alarmbereichen umfasst, die eine Partition des Alarmbereichs bilden; und
der Computer (30) dazu ausgelegt ist, eine Vielzahl von Benachrichtigungen bereitzustellen, wobei jede Benachrichtigung einem Teilsatz des Alarmbereichs entspricht, wobei eine erste Benachrichtigung für einen ersten Teilsatz des Alarmbereichs sich von einer zweiten Benachrichtigung für einen zweiten Teilsatz des Alarmbereichs unterscheidet.

8. Ophthalmisches chirurgisches System (10) nach Anspruch 7, wobei die erste Benachrichtigung eine erste visuelle Charakteristik aufweist, die sich von einer zweiten visuellen Charakteristik der zweiten Benachrichtigung unterscheidet.

9. Ophthalmisches chirurgisches System (10) nach Anspruch 7, wobei die erste Benachrichtigung eine erste Audiocharakteristik aufweist, die sich von einer zweiten Audiocharakteristik der zweiten Benachrichtigung unterscheidet.

10. Ophthalmisches chirurgisches System (10) nach Anspruch 7, wobei:
ein Teilsatz von Alarmbereichen der Vielzahl von Teilsätzen von Alarmbereichen ein beendigender Alarmbereich ist; und
der Computer (30) dazu ausgelegt ist, den chirurgischen Eingriff in Reaktion auf Bestimmen, dass sich die Bewegung des Markers in dem beendigenden Alarmbereich befindet, zu beenden.

11. Ophthalmisches chirurgisches System (10) nach Anspruch 7, wobei:
das ophthalmische chirurgische System (10) ferner einen Anzeigebildschirm umfasst; und
der Computer (30) ausgelegt ist zum:
Anzeigen eines Benachrichtigungssymbols, das eine Vielzahl von Alarmniveaus umfasst, auf dem Anzeigebildschirm, wobei mindestens ein Alarmniveau einem Teilsatz des Alarmbereichs entspricht; und
visuelles Hervorheben des mindestens einen Alarmniveaus in Reaktion auf Bestimmen, dass sich die Bewegung des Markers in dem entsprechenden Teilsatz des Alarmbereichs befindet.

12. Ophthalmisches chirurgisches System (10) nach Anspruch 1, wobei der Computer (30) ferner dazu ausgelegt ist, den Alarmbereich zu bestimmen, der einem ausgewählten chirurgischen Eingriff von einer Vielzahl von chirurgischen Eingriffen entspricht.

13. Ophthalmisches chirurgisches System (10) nach Anspruch 12, wobei:
der ausgewählte chirurgische Eingriff ein Lentikel-Extraktionseingriff ist; und
der Alarmbereich einen maximal akzeptablen Abstand von 50 bis 150 Mikrometer aufweist.

14. Ophthalmisches chirurgisches System (10) nach Anspruch 12, wobei:
der ausgewählte chirurgische Eingriff ein Flap-Erzeugungseingriff ist; und
der Alarmbereich einen maximal akzeptablen Abstand von 100 bis 500 Mikrometer aufweist.

15. Ophthalmisches chirurgisches System (10) nach Anspruch 12, wobei:
der ausgewählte chirurgische Eingriff ein Kataraktentfernungseingriff ist; und
der Alarmbereich einen maximal akzeptablen Abstand von 100 bis 500 Mikrometer aufweist.

## Revendications

1. Système chirurgical ophtalmique (10) pour créer un marqueur dans une cornée d'un œil (22) pour une procédure chirurgicale parmi une pluralité de procédures chirurgicales, comprenant :
une pluralité de composants contrôlables comprenant :
une source laser (12) configurée pour générer un faisceau laser ayant une pluralité d'impulsions ultra-courtes ;
un dispositif de balayage (16) configuré pour diriger transversalement et longitudinalement un point focal du faisceau laser ;
un objectif (18) configuré pour focaliser le point focal à travers une interface patient (20) vers l'œil (22) ; et
une caméra (38) configurée pour imager le mouvement de l'œil (22) ; et
un ordinateur (30) configuré pour :
identifier le marqueur correspondant à la procédure chirurgicale, les procédures chirurgicales comprenant une intervention d'extraction de la cataracte, le marqueur comprenant un marqueur qui délimite une capsule de cristallin de l'œil (22) ;
créer le marqueur dans la cornée qui délimite la capsule de cristallin de l'œil (22), le marqueur ayant une forme qui peut indiquer le mouvement de rotation de l'œil, par :
l'ordre au dispositif de balayage (16) de diriger transversalement et longitudinalement le point focal vers une région périphérique de la cornée ;
l'ordre à un ou plusieurs des composants contrôlables de créer le marqueur dans la région périphérique de la cornée ;
déterminer que le mouvement du marqueur se trouve dans une plage d'alerte, la plage d'alerte indiquant une quantité inacceptable de mouvement du marqueur ; et
fournir une ou plusieurs notifications en réponse à la détermination que le mouvement du marqueur se trouve dans la plage d'alerte.

2. Système chirurgical ophtalmique (10) selon la revendication 1, la forme du marqueur étant choisie parmi une ou plusieurs des formes suivantes : un polygone, une ligne, une pluralité de lignes, une pluralité de lignes se coupant en un point, une pluralité de lignes se coupant en une pluralité de points, un cercle avec une ligne, un ovale et un ou plusieurs caractères alphanumériques.

3. Système chirurgical ophtalmique (10) selon la revendication 1,
une autre procédure chirurgicale parmi la pluralité de procédures chirurgicales étant une procédure d'extraction de lenticule ; et
l'ordinateur (30) étant configuré pour :
identifier un marqueur qui est externe et proche d'une limite extérieure d'un lenticule à extraire ; et
créer le marqueur externe et proche de la limite extérieure.

4. Système chirurgical ophtalmique (10) selon la revendication 1,
une autre procédure chirurgicale parmi la pluralité de procédures chirurgicales étant une procédure de création de lambeau ; et
l'ordinateur (30) étant configuré pour :
identifier un marqueur qui est externe et proche d'une limite extérieure d'un lambeau à créer ; et
créer le marqueur externe et proche de la limite extérieure.

5. Système chirurgical ophtalmique (10) selon la revendication 1, une notification parmi la ou les notifications étant une notification audio.

6. Système chirurgical ophtalmique (10) selon la revendication 1, une notification parmi la ou les notifications étant une notification visuelle.

7. Système chirurgical ophtalmique (10) selon la revendication 1,
la plage d'alerte comprenant une pluralité de plages d'alerte de sous-ensemble qui ne se chevauchent pas et qui forment une partition de la plage d'alerte ; et
l'ordinateur (30) étant configuré pour fournir une pluralité de notifications, chaque notification correspondant à un plage d'alerte de sous-ensemble, une première notification pour une première plage d'alerte de sous-ensemble étant distincte d'une deuxième notification pour une deuxième plage d'alerte de sous-ensemble.

8. Système chirurgical ophtalmique (10) selon la revendication 7, la première notification ayant une première caractéristique visuelle qui est distincte d'une deuxième caractéristique visuelle de la deuxième notification.

9. Système chirurgical ophtalmique (10) selon la revendication 7, la première notification ayant une première caractéristique audio qui est distincte d'une deuxième caractéristique audio de la deuxième notification.

10. Système chirurgical ophtalmique (10) selon la revendication 7,
une plage d'alerte de sous-ensemble parmi la pluralité de plages d'alerte de sous-ensemble étant une plage d'alerte de fin ; et
l'ordinateur (30) étant configuré pour mettre fin à la procédure chirurgicale en réponse à la détermination que le mouvement du marqueur se trouve dans la plage d'alerte de fin.

11. Système chirurgical ophtalmique (10) selon la revendication 7,
le système chirurgical ophtalmique (10) comprenant en outre un écran d'affichage ; et
l'ordinateur (30) étant configuré pour :
afficher une icône de notification comprenant une pluralité de niveaux d'alerte sur l'écran d'affichage, au moins un niveau d'alerte correspondant à une plage d'alerte de sous-ensemble ; et
mettre en évidence visuellement l'au moins un niveau d'alerte en réponse à la détermination que le mouvement du marqueur se trouve dans la plage d'alerte de sous-ensemble correspondante.

12. Système chirurgical ophtalmique (10) selon la revendication 1,
l'ordinateur (30) étant en outre configuré pour déterminer la plage d'alerte correspondant à une procédure chirurgicale sélectionnée parmi la pluralité de procédures chirurgicales.

13. Système chirurgical ophtalmique (10) selon la revendication 12,
la procédure chirurgicale sélectionnée étant une procédure d'extraction de lenticule ; et
la plage d'alerte ayant une distance maximale acceptable de 50 à 150 microns.

14. Système chirurgical ophtalmique (10) selon la revendication 12,
la procédure chirurgicale sélectionnée étant une procédure de création de lambeau ; et
la plage d'alerte ayant une distance maximale acceptable de 100 à 500 microns.

15. Système chirurgical ophtalmique (10) selon la revendication 12,
la procédure chirurgicale sélectionnée étant une procédure d'extraction de la cataracte ; et
la plage d'alerte ayant une distance maximale acceptable de 100 à 500 microns.
